Europäisches Patentamt

European Patent Office

Office européen des brevets

(11) Publication number: 0 403 824
A2

## EUROPEAN PATENT APPLICATION

(21) Application number: 90109917.6

(22) Date of filing: 24.05.90

(51) Int. Cl.5: A61B 5/00

(30) Priority: 23.06.89 JP 161919/89

(43) Date of publication of application:
27.12.90 Bulletin 90/52

(84) Designated Contracting States:
DE FR GB IT

(71) Applicant: FUKUDA DENSHI CO., LTD.
39-4, Hongo 3-chome Bunkyo-ku
Tokyo 113(JP)

(72) Inventor: Higuma, Shingo, c/o Fukuda Denshi
Co. Ltd.
Hongo Enterprise Place, 2-35-8 Hongo
Bunkyo-ku, Tokyo(JP)

(74) Representative: Behn, Klaus, Dipl.-Ing.
Patentanwalt Lindenberg 34
D-8134 Pöcking bei München(DE)

(54) Method for treating keis and method for controlling remotely the treatment of keis.

(57) A method for treating keis by which the living body signal of a patient is dealed.

The method has the steps of inputting keis with specific name, generating key cords corresponding respectively to the keis, and treating the contents of key functions corresponding respectively to the key cords.

Moreover, a method for controlling remotely the treatment of keis.

The method has the steps of inputting keis with specific names into a first apparatus, outputting key cords corresponding respectively to the keis from the first apparatus, transmitting the key cords to a second apparatus, and treating the content of keis functions corresponding respectively to the key cords, in the second apparatus.

## METHOD FOR TREATING KEIS AND METHOD FOR CONTROLLING REMOTELY THE TREATMENT OF KEIS

### BACKGROUND OF THE INVENTION

#### 1. Field of the invention

The present invention relates to a method for treating keis and a method for controlling remotely the treatment of keis.

More particularly, it relates to a method for treating keis and a method for controlling remotely the treatment of keis ,in order to deal a living body signal in an apparatus for monitoring a patient.

#### 2. Description of the Related Art

Geneally, a living body signal is treated in an apparatus for monitoring a patient, as follows.

That is to say, the living body signal may be treated by means of a method, wherein when a key, of which name is given, is pushed, it is input in the apparatus for monitoring a patient, and accordingly a key cord corresponding to the key is generated, thereby a function corresponding to the key cord is treated.

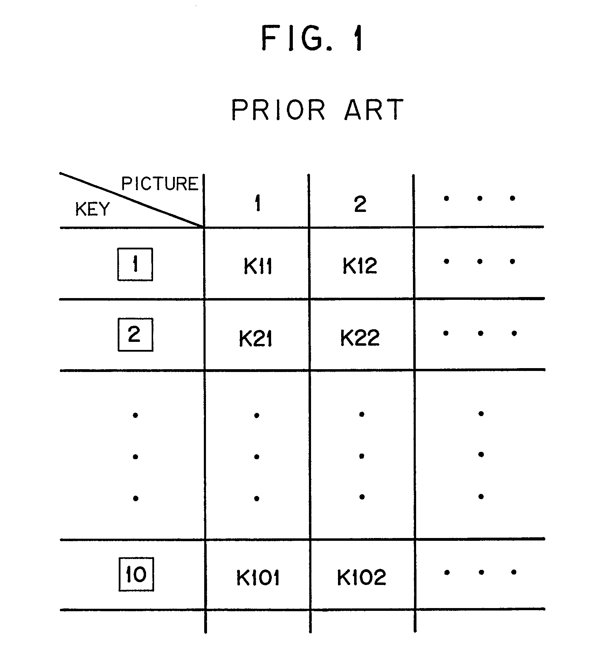

Figure 1 is an explanatory drawing of the conventional method, which is tabulated.

In a table of Fig. 1, reference numerals 1 , 2 • • • 10 in a perpendicular colum show keis , and reference numerals 1, 2 • • • in a transverse column show pictures

In Fig. 1, for example, when a key 1 is input, a function K11 is treated in case of a picture 1, and a function K 12 is treated in case of a pecture 2.

With respect to another keis 2, 3 • • • 10, treatments are done in the same way as with respect to the key 1.

In the conventional method as shown in Fig. 1, one key dose not correspond to one function.

That is to say, as the number of key is restrected, for example 10, in the prior art, one key has many different functions in accordance with different pictures.

For example, though the key 1 is input, the function K11 is treated regarding the picture 1, and the function K12 is treated regarding the picture 2, as shown in Fig, 1.

As hereinbefore, in the conventional method, one key does not correspond to one function.

Hence, the plan of the above conventinal method is very complex, and the operation of the key of the same method is also trouble some.

Moreover, a key has been input and it has been treated, conventionally, by a method for controlling remotely the treatment of keis between two apparatuses, for example, a bedside monitor and a center.

In the above remote control metod, the key has been input to the bedside monitor and it has been treated in the same monitor, by operating remotely in the center, in stead of inputting directly the key in the bedside monitor.

In this case, there has been the above inconvenience between the bedside monitor and the center, that is to say, one key does not correspond to one function.

Accordingly, with respect to the conventional remote control method, the plan thereof is also very complex and the operation thereof is troublesome.

### SUMMARY OF THE INVENTION

An object of the present invention is to simplify a method for treating keis and a method for controlling remotely the treatment of keis.

The above-mentioned object can be achieved by a method for treating keis by which the living body signal of a patient is dealed, comprising the steps of inputting keis with specific name, generating key cords corresponding respectively to said keis, and treating the contents of key functions corresponding respectively to said key cords, and a method for controlling remotely the treatment of keis, comprising the steps of inputting keis with specific names into a first apparatus, outputting key cords corresponding respectively to said keis from said first apparatus, transmitting said key cords to second apparatus, and treating the content of keis functions corresponding respectively to said key cords, in said second apparatus.

### BRIEF DESCRIPTION OF THE DRAWINGS

Further features and advantages of the present invention will be apparent from the ensuring dencription with reference to the accompaning drawings, wherein:

Fig. 1 is an explanatory drawing of the conventional method for treating keis;

Fig. 2A and 2B are drawings of the principle of the present invention, and,

Fig. 3A and 3B are drawings of embodiments of the present invention.

### DESCRIPTION OF THE PREFERRED EMBODIMENTS

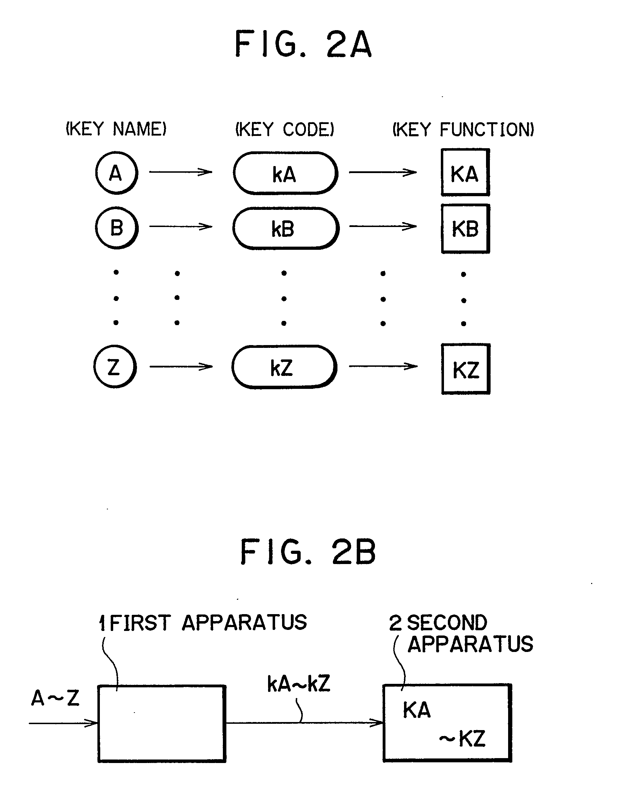

Figure 2A is a drawing of the principle of a method for treating keis in accordance with the present invention

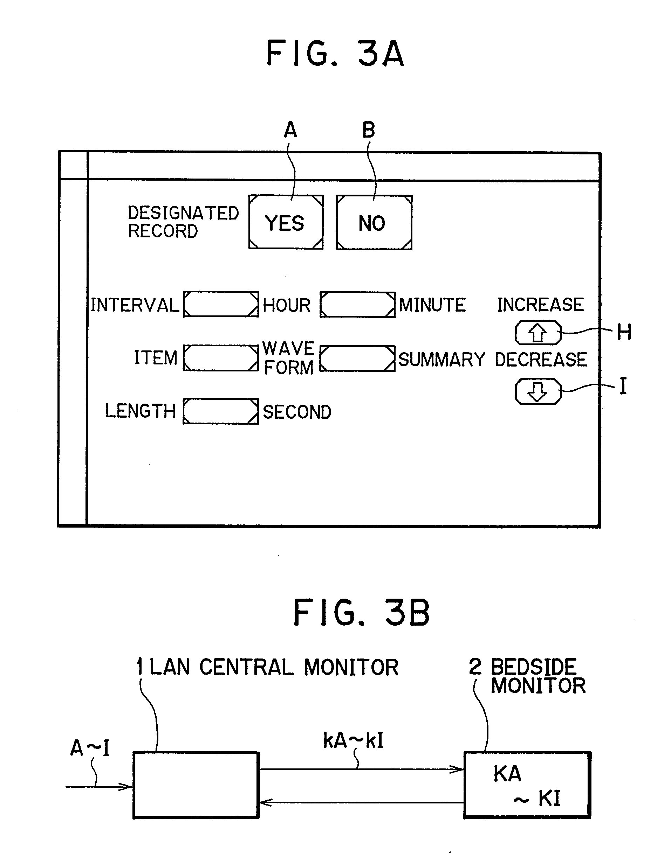

Figure 3A is a drawing of an embodiment of the same method.

The enbodiment as shown in Fig. 3A may be carried out according to the principle as shown in Fig. 2A.

In Fig. 2A, reference numerals A, B • • • Z show key names, kA, kB . . . kZ key cords, and KA, KB • • • KZ key functions.

The key name A is a specific name, for example, "RECORD".

The key name A corresponds to the key code kA, which key cods KA corresponds to the key function KA.

Hence, when a key, of which name is "A", is input in an apparatus for monitoring a patient, for example, a bedside monitor, the key code kA is always generated in the bedside monitor, and according to the key code kA, the key function KA is always carried out, in the bedside monitor.

Like the key name A, the key code kA, and the key function KA, the key names B, C • • • Z correspond, to the key codes kB, kC • • • kZ, each, which key codes kB, kC • • • kZ correspond to the key functions KB, KC • • • KZ, respectively.

That is to say, according to a method for treating keis in accordance with the present invention, when a key having a specific name is input, a key code corresponding only to the specific name is generated and the content of the function corresponding to only the key code is treated.

The above method of the present invention will be explained more concretely with reference to Fig. 3A.

Fig. 3A shows many keis A to I displayed on the picture of the Braun tube (the cathode-ray tube) of the bedside monitor, in the case where the living body signal of a patient will be recorded on a recording paper, in the range previously disignated.

At first, keis A and B have been already displayed respectively on the picture, with respect to a term "DESIGNATED RECORD", which term means whether or not the living body signal of a patient may be recorded on a recording peper in the range previously desgnated.

A word YES" of the key A means that the above living body signal should be recorded.

On the contrary, a word "NO" of the key B means that the same signal should not be recorded.

When the key A is pushed on the picture, a signal corresponding to the key A is input into the bedside monitor.

Accordingly, the key code kA is generated, and the key function KA corresponding to the key code kA may be treated.

The result is that keis will be displayed on the picture, as follows.

That is to say, with respect to a word "INTERVAL", keis C and D are displayed respectively, with yespect to a word "TEM", keis E and F are displayed respectively, with respect to a word "LENGTH", a key G is displayed, with respect to the keis C, D and G , keis H and I are displayed respectively.

As apparently from the explanation hereinbefore, the key function KA as shown in Fig. 2A, is a function that the keis C to I are displayed on the picture, as shown in Fig. 3A.

The word "INTERVAL" means the interval of the time when the living body signal should be recorded on the recording paper.

The key C is a key by which the above interval of the time may be designated to 24 hours.

The key D is a key by which the above interval of the time may be designated to 60 minutes.

That is to say, the key functions KC and KD, as shown in Fig. 2A, are functions that the interval of the times may be designated by keis C and D1 respectively, as shown in Fig. 3A.

The key E is a key by which the wave form of the living body signal may be recorded.

The key F is a key by which the numerical value regarding the living body signal may be recorded.

That is to say, the key functions HE and KF, as shown in Fig. 2A, are functions that the wave form and numerical value may be recorded by the keis E and F, respectively, as shown in Fig. 3A.

The word "LENGTH" means the time during which the recording paper may be moved.

The key G is a key by which the above recording paper moving time may be designated to 99 seconds.

That is to say, the key function KG, as shown in Fig. 2A, is a function that this moving time may be designated by the key G as shown in Fig. 3A.

The key H is a key by which the numerical value of the content of the key C, D, or G may be increased.

The key I is a key by which the numerical value of the content of the key C, D or G may be decresed.

That is to say, the key functions KH and KI, as shown in Fig. 2A, are functions that the numerical value may by increased and decreased by the keis H and I, respectively, as shown in Fig. 3A.

As explained bereinbefore, the functions KC to KI corresponding to the keis C to 1 may be treated.

Figure 2B is a drawing of the principle of a method for controlling remotely the treatment of keis.

Figure 3B is a drawing of an embodiment of the same system.

In Fig. 2B, reference numeral 1 shows a first apparatus, and 2 a second apparatus.

Fig. 2B shows a method wherin the first apparatus 1 controls remotely the second system 2.

The first apparats 1 is an apparatus in which when keis A to Z with specific names are input, key codes kA to kZ corres ponding to the keis A to 2, respectively, are output.

The second apparatus 2 is an apparatus in which when the key cords kA to kZ are input, key functions HA to HZ corresponding to the key cords kA to kZ, respectively, are treated.

The keis A to Z corresponds to the KEY NAMES A to Z, the key cords kA to kZ does to the KEY CODES kA to kZ, and the key functions KA to KZ does to KEY FUNCTIONS KA to KZ, respectively, as shown in Fig. 2A.

Fig. 3B shows more concretely Fig. 2B.

In Fig. 3B, the first apparatus 1 is a LAN central monitor, for example, and the second apparatus 2 is a bedside monitor, fcr example.

The relationship between the LAN central monitor 1 and the bedside monitor 2 is as follows.

That is to say, the LAN central monitor 1 is a central monitor connected with the bedside monitor 2 though LAN (Local Area Network).

As well known, according to LAN, not only the numerical value of the living body signal, but also the wave form of it may be transmitted in digital, and the high speed and mutual direction communication may be done.

When keis A to I are input into the LAN central monitor 1, which keis A to I correspond to the keis A to I as shown in Fig. 3A, key cords kA to kI are output and are transmitted to the bedside monitor 2 through LAN as aforementioned.

When the key kA to kI cords are input into the bedside monitor 2, the key functions KA to KI are treated in it.

According to the above remote control method, the same effect may be obtained as if the keis A to I are input directly in to the bedside monitor 2.

After the keis function KA to KI are treated, all the states 5 are transmitted from the bedside monitor 2 to the LAN central monitor 1 through LAN.

All the states S include not only the content of the changed state, but also the content of the unchanged state.

According to the present invention, a method or treating keis and a method for controlling remotely the treatment of keis have been offered, as shown in Fig. 2A, 3A, 2B and 3B.

The key names A to 2, the key codes kA to k2, and the key functions KA to KZ have been capable of corresponding to each other, as shown in Fig. 2A.

Hence, because the content of the key functions are treated by the keis with an one to one correspondence, a method for treating keis may be remarkably simplified regarding the plan and operation thereof.

Moreover, if the keis with an one to one correspondence are only input to the first apparatus 1, the content of the key functions may be treated remotely in the second appartus 2, as shown in Fig. 2B.

Thereby, a method for controlling remotely the treatment of keis has been also very simplified.

## Claims

1. A method for treating keis by which the living body signal of a patient is dealed, comprising;
the steps of inputting keis with specific name, generating key cords corresponding respectively to said keis, and
treating the contents of key functions corresponding respectively to said key cords.

2. A method for controlling remotely the treatment of keis, comprising;
the steps of inputting keis with specific names into a first apparatus,
outputting key cords corresponding respectively to said keis from said first apparatus,
transmitting said key cords to a second apparatus, and
treating the content of keis functions corresponding respectively to said key cords, in said second apparatus.

3. A method for controlling remotely the treatement of keis according to claim 2, wherein
the steps of inputting keis with specitic names into a first apparatus,
outputting key cords correspondig respectively to said keis from said first apparatus,
transmitting said key cords to a second apparatus,
treating the content of keis fuuctions corresponding respectively to said key cords, in said second apparatus, and
transmitting all the states of keis after treated from said secondapparatus to said first apparatus.

4. A method for controlling remotely the treatment of keis according to claim 2, wherein said first apparatus is a LAN central monitor, and said second apparatus is a bedside monitor.

# FIG. 1

## PRIOR ART

| KEY \ PICTURE | 1 | 2 | • • • |
|:---:|:---:|:---:|:---:|
| 1 | K11 | K12 | • • • |
| 2 | K21 | K22 | • • • |
| • • • | • • • | • • • | • • • |
| 10 | K101 | K102 | • • • |

# FIG. 2A

| (KEY NAME) | (KEY CODE) | (KEY FUNCTION) |

# FIG. 2B

# FIG. 3A

# FIG. 3B